(19) Europäisches Patentamt / European Patent Office / Office européen des brevets

(11) **EP 4 172 933 B1**

(12) **FASCICULE DE BREVET EUROPEEN**

(45) Date de publication et mention
de la délivrance du brevet:
**31.07.2024   Bulletin 2024/31**

(21) Numéro de dépôt: **21745361.2**

(22) Date de dépôt: **24.06.2021**

(51) Classification Internationale des Brevets (IPC):
**G06T 7/00** *(2017.01)*      **A61B 34/10** *(2016.01)*

(52) Classification Coopérative des Brevets (CPC):
**G06T 7/0012; A61B 5/055; A61B 34/10;**
A61B 2034/107; G01R 33/5608; G01R 33/56341

(86) Numéro de dépôt international:
**PCT/FR2021/051168**

(87) Numéro de publication internationale:
**WO 2021/260334 (30.12.2021 Gazette 2021/52)**

(54) **DISPOSITIF D'IMAGERIE TUMORALE**

TUMORBILDGEBUNGSVORRICHTUNG

TUMOUR IMAGING DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **26.06.2020   FR 2006763**

(43) Date de publication de la demande:
**03.05.2023   Bulletin 2023/18**

(73) Titulaire: **Institut National de Recherche en Informatique et
en Automatique
78153 Le Chesnay Cedex (FR)**

(72) Inventeurs:
  • **VIGNON-CLEMENTEL, Irène**
    **78153 Le Chesnay Cedex (FR)**
  • **DRASDO, Dirk**
    **78153 Le Chesnay Cedex (FR)**
  • **YIN, Yi**
    **78153 Le Chesnay Cedex (FR)**

(74) Mandataire: **Vidon Brevets & Stratégie
16B, rue de Jouanet
BP 90333
35703 Rennes Cedex 7 (FR)**

(56) Documents cités:
  • **MATSUMOTO RYUTARO ET AL: "Needle Insertion Path Planning System for Lower Abdominal Insertion Based on CT Images", 2018 IEEE INTERNATIONAL CONFERENCE ON ROBOTICS AND BIOMIMETICS (ROBIO), IEEE, 12 December 2018 (2018-12-12), pages 182 - 185, XP033529500, DOI: 10.1109/ROBIO.2018.8664883**
  • **PONDMAN KIRSTEN M ET AL: "MR-Guided Biopsy of the Prostate: An Overview of Techniques and a Systematic Review", EUROPEAN UROLOGY, vol. 54, no. 3, 12 June 2008 (2008-06-12), pages 517 - 527, XP029857550, ISSN: 0302-2838, DOI: 10.1016/J.EURURO.2008.06.001**

EP 4 172 933 B1

## Description

La présente invention concerne le domaine de l'imagerie médicale, et plus particulièrement de l'imagerie des tumeurs. L'imagerie des tumeurs regroupe les méthodes d'analyse non-invasive de tumeurs. Les analyses peuvent viser à déterminer l'étendue des cellules cancéreuses au sein de la tumeur. L'imagerie des tumeurs est réalisée par imagerie par résonnance magnétique (IRM), et en particulier par imagerie par résonnance magnétique de diffusion. Cependant, ces deux techniques d'imagerie ne permettent pas à ce jour d'obtenir d'information précise sur une tumeur dans le corps d'un patient comme une distribution absolue de cellules cancéreuses ou la charge tumorale (« *tumor load* » en anglais). Par charge tumorale, on entend ici le nombre total de cellules de la tumeur, tous types confondus ou chaque type individuellement, en particulier du type cellules cancéreuses.

On connaît l'usage de biopsies comme méthode invasive en complément ou en remplacement de l'imagerie des tumeurs. Une biopsie consiste en un prélèvement localisé de tissus par une aiguille de biopsie. La biopsie permet d'obtenir des informations locales sur la tumeur qui sont inaccessibles par des techniques non-invasives. La biopsie est cependant un prélèvement local, et le tissu prélevé n'est généralement pas représentatif de la tumeur étudiée. La biopsie est en outre un acte chirurgical invasif, et présente des risques médicaux tels qu'une augmentation de la tumeur, une diffusion des cellules cancéreuses dans des tissus sains, un saignement, une infection ou une atteinte nerveuse.

La publication "Needle Insertion Path planning System for Lower Abdominal Insertion Based on CT Images", Matsumoto Ryutaro et al., 2018 International Conférence on Robotics and Biomimetics, pp. 182-185, divulgue une méthode préopérative pour déterminer l'angle et la profondeur pour l'insertion d'une aiguille.

La publication "MR-Guided Biopsy of the Prostate: An Overview of Techniques and a Systematic Review", Pondman Kirsten M et al., European Urology, vol. 54, no. 3, pp. 517-527, divulgue l'état de la technique pour la biopsie de la prostate guidée par imagerie à résonance magnétique.

Il n'existe pas de méthode permettant de déterminer un emplacement représentatif pour effectuer une biopsie. De ce fait, il est actuellement nécessaire de réaliser beaucoup de biopsies, parfois près d'une dizaine, pour augmenter les chances d'obtenir assez d'informations pour déterminer des informations sur la distribution absolue recherchée ou la charge tumorale.

L'invention vient améliorer la situation. A cet effet l'invention propose un dispositif d'imagerie comprenant :

- une mémoire, agencée pour recevoir

    des données d'imagerie comprenant au moins une image matricielle tirée d'une image par résonnance magnétique de diffusion en coupe d'une tumeur, chaque pixel de ladite image matricielle étant associé à un paramètre de diffusion dont la valeur représente la mobilité de molécules d'eau au sein de ladite tumeur,
    des données de biopsie comprenant des données d'aiguille définissant les dimensions d'une ponction réalisée au moyen d'une aiguille de biopsie, et
    des données de procédure comprenant un nombre de ponctions à effectuer dans une procédure de biopsie supérieur ou égal à 2 et un jeu de contraintes associées à des contraintes interventionnelles à respecter pour réaliser le nombre de ponctions à effectuer,

- un suréchantillonneur, agencé pour suréchantillonner l'image matricielle en une image de traitement dont la résolution spatiale est telle que chaque pixel de l'image de traitement correspond à une région de l'image en coupe sensiblement carrée dont le côté présente une longueur inférieure à une dimension de diamètre d'aiguille des données d'aiguille,
- un découpeur agencé pour partitionner l'image de traitement issue du suréchantillonneur en un jeu de régions dans lequel chaque région

    comprend des pixels de l'image de traitement directement voisins au moins deux à deux,
    est associée à un paramètre de diffusion dont la valeur est sensiblement égale à la moyenne des valeurs des paramètres de diffusion associés aux pixels qui la composent,
    la variance des valeurs des paramètres de diffusion associés aux pixels de la région est inférieure à une valeur de variance donnée,
    correspond à une portion de l'image par résonnance magnétique de diffusion en coupe de ladite tumeur dont les dimensions sont supérieures ou sensiblement égales aux dimensions d'une ponction réalisée au moyen d'une aiguille de biopsie des données de biopsie,

    et dans lequel les paramètres de diffusion associés à chaque région du jeu de régions forment un jeu de paramètres de diffusion,
- un sélecteur, agencé pour

déterminer au sein du jeu de paramètres de diffusion un sous-jeu de paramètres de diffusion comprenant autant de paramètres de diffusion que le nombre de ponctions à effectuer des données de procédure, le sous-jeu de paramètres de diffusion comprenant au moins un premier paramètre de diffusion choisi dans le premier décile des valeurs de paramètre de diffusion du jeu de paramètres de diffusion, un deuxième paramètre de diffusion dans le dernier décile des valeurs de paramètre de diffusion du jeu de paramètres de diffusion, et, lorsque le nombre de ponctions à effectuer des données de procédure est supérieur à 2, des paramètres de diffusion dont les valeurs de paramètre de diffusion respectives sont comprises entre la valeur du premier paramètre de diffusion et la valeur du deuxième paramètre de diffusion,

renvoyer un sous-jeu de régions tirées du jeu de régions et dans lequel chaque région est associée à un paramètre de diffusion sensiblement égal à l'un des paramètres du sous-jeu de paramètres de diffusion,

- un guide, agencé pour déterminer, à partir du sous-jeu de régions, des données de jeux de paramètres de ponction dont le nombre est égal au nombre de ponctions à effectuer des données de procédure, chaque jeu de paramètres de ponction comprenant une profondeur de ponction, une orientation de ponction et un point d'entrée de ponction et définissant avec les données d'aiguille une zone de ponction qui est sensiblement incluse dans une des régions du sous-jeu de régions, de telle sorte que les jeux de paramètres de ponction respectent ensemble le jeu de contraintes des données de procédure .

Ce dispositif d'imagerie est très avantageux, car il permet de déterminer de manière automatique un nombre réduit d'emplacements de biopsie garantissant des informations pertinentes. En effet, les ponctions déterminées par le dispositif d'imagerie sont bien plus représentatives de l'ensemble de la tumeur que des ponctions prises aléatoirement, tout en étant moins nombreuses.

Dans diverses variantes, le dispositif peut présenter une ou plusieurs des caractéristiques suivantes :

- le suréchantillonneur est agencé pour suréchantillonner l'image matricielle en l'image de traitement en réalisant une interpolation,
- le suréchantillonneur est agencé pour réaliser une interpolation bicubique pour suréchantillonner l'image matricielle en l'image de traitement,
- le découpeur est agencé pour partitionner l'image de traitement issue du suréchantillonneur par une méthode de superpixelisation,
- le nombre de ponctions à effectuer dans une procédure de biopsie des données de procédure que stocke la mémoire est inférieur ou égal à 4,
- le sélecteur choisit le premier paramètre de diffusion dans le deuxième percentile des valeurs de paramètre de diffusion du jeu de paramètres de diffusion, et le deuxième paramètre de diffusion dans le quatre-vingt-dix-huitième percentile des valeurs de paramètre de diffusion du jeu de paramètres de diffusion,
- le premier paramètre de diffusion et le deuxième paramètre de diffusion sont chacun égaux à la valeur médiane de leur quantile respectif,
- lorsque le nombre de ponctions à effectuer des données de procédure est supérieur à 2, le sélecteur choisit les paramètres de diffusion du sous-jeu de paramètres de diffusion de sorte qu'ils soient équidistants deux à deux,
- lorsque le nombre de ponctions à effectuer des données de procédure est supérieur à 2, le sélecteur détermine une valeur médiane des paramètres de diffusion du jeu de paramètres de diffusion, et détermine les paramètres de diffusion, j étant un entier naturel compris entre 1 et le nombre de ponctions à effectuer des données de procédure, du sous-jeu de paramètres de diffusion selon l'équation :

$$Dj = Dini + Delta(j-1),$$

où *Dini* vérifie l'équation :

$$Dini = M - min(|M - Dmin|, |M - Dmax|)$$

et *Delta* vérifie l'équation :

$$Delta = 2(M - Dini)/(N-1),$$

*Dmin* étant égal à la valeur du premier paramètre de diffusion déterminé par le sélecteur et *Dmax* étant égal à la valeur du deuxième paramètre de diffusion du sous-jeu de paramètres de diffusion déterminé par le sélecteur,

- la profondeur de ponction, l'orientation de ponction, le point d'entrée de ponction de chacun des jeux de paramètres de ponction déterminés par le guide définissent avec les données de biopsie une trajectoire de ponction de l'aiguille de biopsie, et dans lequel le jeu de contraintes des données de procédure stockées dans la mémoire comprend une ou plusieurs contraintes choisies dans le groupe comprenant :

  - les ponctions d'une procédure de biopsie doivent avoir le même point d'entrée dans la tumeur,
  - les trajectoires de ponction sont en ligne droite et doivent être comprises dans un angle maximal de ponction,
  - la profondeur de ponction est inférieure à une profondeur maximale de ponction,
  - la zone de ponction est éloignée des bords de la tumeur d'une distance prédéterminée,
  - si la tumeur comprend une zone nécrosée, la trajectoire de ponction ne pénètre pas ladite zone nécrosée,
  - si la tumeur comprend une zone de tissus gras, aucune des zones de ponction n'intersecte ladite zone de tissus gras,
  - au moins un des points d'entrée des données de jeux de paramètres de ponction est fixé à l'avance,

- la mémoire est en outre agencée pour recevoir des données de ponction comprenant un jeu de densités cellulaires, chaque densité cellulaire étant associée à l'une des zones de ponction des données de jeux de paramètres de ponction, chaque densité cellulaire ayant une valeur représentant une densité cellulaire de ladite zone de ponction, le dispositif d'imagerie comprenant en outre :

  - un corrélateur, agencé pour associer à chaque valeur de densité cellulaire associée à l'une des zones de ponction la valeur de paramètre de diffusion de la région dans laquelle est sensiblement incluse ladite zone de ponction, et ce pour le jeu de région, et pour en tirer des données de corrélation associant des valeurs de densité cellulaire et des valeurs de paramètre de diffusion parmi le jeu de paramètres de diffusion, et
  - un constructeur, agencé pour déterminer des données de densité à partir de l'image de traitement et des données de corrélations, les données de densité comprenant une image de densité dont chaque pixel, qui correspond à une région de l'image en coupe, est associé à une densité cellulaire dont la valeur est associée dans les données de corrélation à la valeur de paramètre de diffusion d'un pixel de l'image de traitement correspondant à ladite région de l'image en coupe dudit pixel.

- le corrélateur réalise une interpolation linéaire ou non linéaire strictement monotone sur la base de paires formées d'une valeur de paramètre de diffusion et d'une valeur de densité cellulaire associées à une zone de ponction et tire du résultat de ladite interpolation les données de corrélation,
- la densité cellulaire des données de ponction et des données de densité correspond à une densité de cellules totale ou une densité de cellules cancéreuses,
- le dispositif comprend en outre un estimateur agencé pour déterminer une charge tumorale de la tumeur sur la base des données de densité .

D'autres caractéristiques et avantages de l'invention seront exposés en détail dans la description ci-après, faite en référence aux dessins annexés, sur lesquels :

- [Fig. 1] représente une vue schématique du dispositif d'imagerie selon l'invention,
- [Fig. 2] représente une image matricielle d'une tumeur obtenue par résonnance magnétique de diffusion reçue par le dispositif d'imagerie de la figure 1,
- [Fig. 3] représente une vue schématique d'un jeu de contraintes reçu par le dispositif d'imagerie de la figure 1,
- [Fig. 4] représente une image de traitement obtenue par le dispositif d'imagerie de la figure 1,
- [Fig. 5] représente un jeu de régions déterminé par le dispositif d'imagerie de la figure 1,
- [Fig. 6] représente un jeu de paramètres de diffusion déterminé par le dispositif d'imagerie de la figure 1,
- [Fig. 7] représente des jeux de paramètres de ponction déterminés par le dispositif d'imagerie de la figure 1,
- [Fig. 8] représente un sous-jeu de régions déterminé par le dispositif d'imagerie de la figure 1,
- [Fig. 9] représente des régions du sous-jeu de régions de la figure 7,
- [Fig. 10] représente un autre sous-jeu de régions déterminé par le dispositif d'imagerie de la figure 1,
- [Fig. 11] représente un détail de la figure 10,
- [Fig. 12] représente une variante du dispositif d'imagerie de la figure 1,
- [Fig. 13] représente une première interpolation linéaire réalisée par le dispositif d'imagerie de la figure 12,
- [Fig. 14] représente une deuxième interpolation linéaire réalisée par le dispositif d'imagerie de la figure 12,
- [Fig. 15] représente une troisième interpolation linéaire réalisée par le dispositif d'imagerie de la figure 12,
- [Fig. 16] représente une estimation de charge tumorale réalisée par le dispositif d'imagerie de la figure 12,

- [Fig. 17] représente des charges tumorales de la figure 16 déterminées par le dispositif d'imagerie de la figure 12,
- [Fig. 18] représente des charges tumorales mesurées pour des ponctions prises aléatoirement en respectant le jeu de contraintes.

Les dessins annexés contiennent, pour l'essentiel, des éléments de caractère certain. Ils pourront donc non seulement servir à mieux faire comprendre la présente invention, mais aussi contribuer à sa définition, le cas échéant.

La description est suivie d'une Annexe A comprenant des formules mathématiques. Cette annexe fait partie intégrante de la description.

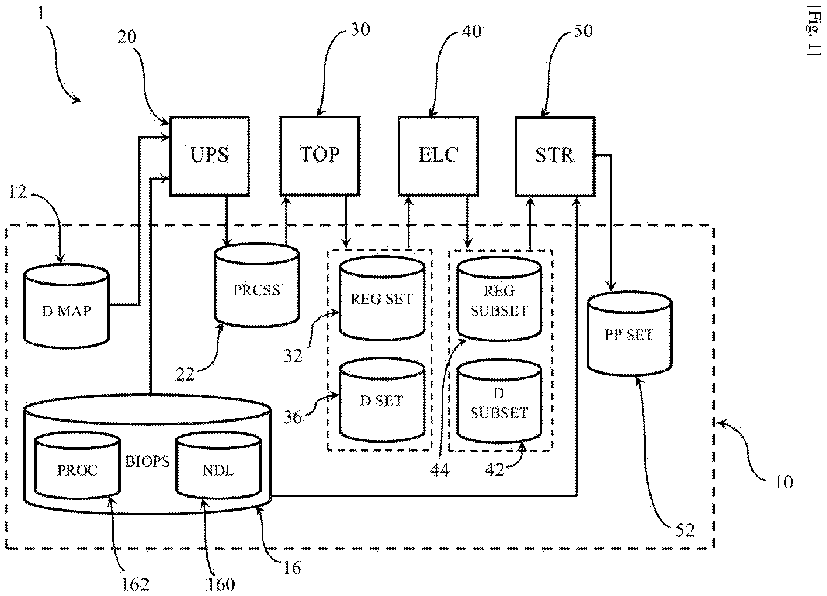

Il est fait référence aux figures 1 à 11.

Un dispositif d'imagerie 1 selon l'invention comprend une mémoire 10, un suréchantillonneur 20, un découpeur 30, un sélecteur 40 et un guide 50.

La mémoire 10 peut être tout type de stockage de données propre à recevoir des données numériques : disque dur, disque dur à mémoire flash (SSD en anglais), mémoire flash sous toute forme, mémoire vive, disque magnétique, stockage distribué localement ou dans le cloud, etc. Les données calculées par le dispositif 1 peuvent être stockées sur tout type de mémoire similaire à la mémoire 10, ou sur celle-ci. Ces données peuvent être effacées après que le dispositif ait effectué ses tâches ou conservées.

Le suréchantillonneur 20, le découpeur 30, le sélecteur 40 et le guide 50 sont ici des programmes exécutés par le processeur de l'ordinateur. En variante, un ou plusieurs de ces éléments pourrait être mis en oeuvre de manière différente au moyen d'un processeur dédié. Par processeur, il doit être compris tout processeur adapté aux traitements de données décrits plus bas. Un tel processeur peut être réalisé de toute manière connue, sous la forme d'un microprocesseur pour ordinateur personnel, d'une puce dédiée de type FPGA ou SoC (« system on chip » en anglais), d'une ressource de calcul sur une grille, d'un microcontrôleur, ou de toute autre forme propre à fournir la puissance de calcul nécessaire à la réalisation décrite plus bas. Un ou plusieurs de ces éléments peuvent également être réalisés sous la forme de circuits électroniques spécialisés tel un ASIC. Une combinaison de processeurs et de circuits électroniques peut également être envisagée.

La mémoire 10 reçoit des données d'imagerie 12, comprenant au moins une image matricielle 14 issue d'une image en coupe d'une tumeur 200 par résonnance magnétique de diffusion (« *Diffusion-weighted magnetic resonance imaging* » en anglais, abrégée en « *Diffusion MRI* », ou « *DWI* »). L'image matricielle 14 est aussi connue sous le nom de « *D-map* ». La résonnance magnétique de diffusion est une technique d'imagerie de tissus consistant à mesurer la diffusion de molécules d'eaux au sein de ces tissus. Chaque pixel de l'image matricielle 14 est ainsi associé à un paramètre de diffusion dont la valeur représente la mobilité de molécules d'eau au sein des tissus de la tumeur 200. Plus la valeur du paramètre de diffusion est élevée, plus la diffusion des molécules d'eau est forte, ou, dit autrement, plus les molécules d'eau sont libres de se déplacer au sein des tissus.

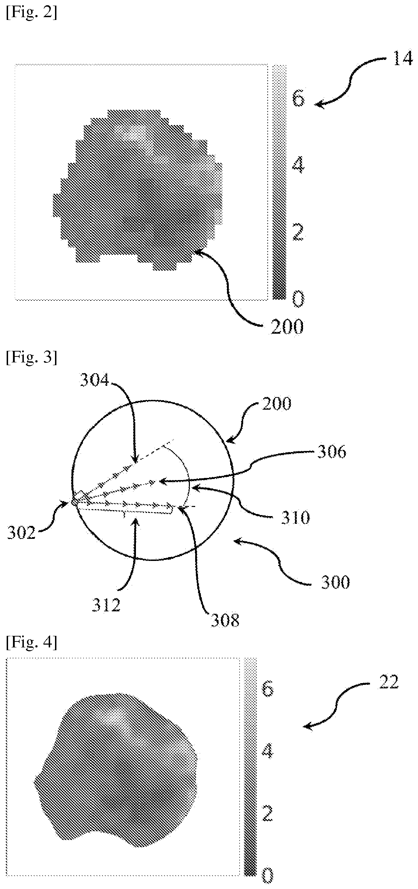

Les valeurs de paramètre de diffusion dépendent beaucoup de l'appareil d'imagerie par résonnance magnétique de diffusion qui les génère, ce qui rend généralement leur analyse impossible sans informations supplémentaires. Dans l'exemple décrit ici, l'image matricielle 14 représentée sur la figure 2 présente des pixels dont le côté correspond à une zone sensiblement carrée de 2,1 mm de côté.

Les travaux de la Demanderesse ont montré que la valeur de diffusion est corrélée à la densité cellulaire. Ainsi, partant d'une image matricielle 14 issue d'une image en coupe par résonnance magnétique de diffusion, il serait en théorie possible d'observer les hétérogénéités cellulaires au sein de la tumeur, au moins qualitativement. Cependant, la résolution des images matricielles fournies par les dispositifs d'imagerie par résonnance magnétique de diffusion actuels est trop faible pour représenter de manière exploitable ces hétérogénéités, de sorte qu'elles ne peuvent pas être utilisées en pratique, sauf à réaliser une tumorectomie complète pour constater la correspondance. Il est bien entendu exclu (et dans la plupart des cas impossible) de réaliser une tumorectomie systématique, d'autant plus que si cela était possible, l'imagerie et les biopsies n'auraient plus aucun intérêt.

Dans un mode de réalisation, les données d'imagerie 12 comprennent plusieurs images matricielles 14 représentant des images en coupe successives de la tumeur 200. Cela permet d'avoir une vue en trois dimensions de la tumeur 200. Dans le mode de réalisation décrit ici, les données d'imagerie 12 comprennent 9 images matricielles 14. Dans un autre mode de réalisation, les données d'imagerie 12 comprennent 40 images matricielles 14 dont sont tirées 9 images matricielles 14 représentant les coupes les plus proches d'un plan médian de la tumeur 200. Une de ces images matricielles 14 est représentée sur la figure 2, l'échelle à droite de la figure indiquant une correspondance entre intensité des pixels et valeur de paramètre de diffusion.

La mémoire 10 reçoit également des données de biopsie 16, comprenant des données d'aiguille 160 et des données de procédure 162.

Les données d'aiguille 160 définissent les dimensions d'une ponction réalisée au moyen d'une aiguille de biopsie. Dans le mode de réalisation décrit ici, les dimensions de la ponction comprennent un diamètre de ponction et une longueur de ponction, et le prélèvement par l'aiguille de biopsie est une zone de ponction sensiblement cylindrique définie par ce diamètre et cette longueur. Dans l'exemple décrit ici, le diamètre de ponction vaut sensiblement 0,84 mm, et la longueur de ponction vaut 4,55 ± 1,45 mm.

Les données de procédure 162 comprennent un nombre de ponctions à effectuer dans une procédure de biopsie. Le nombre de ponctions à effectuer est un nombre entier supérieur ou égal à 2. Il est en effet nécessaire d'effectuer au moins deux ponctions pour obtenir assez d'informations sur la tumeur 200. Dans le mode de réalisation décrit ici, le nombre de ponctions est compris entre 2 et 4, inclus.

Les données de procédure 162 comprennent également un jeu de contraintes 300. Le jeu de contraintes définit des contraintes interventionnelles à respecter dans une procédure de biopsie pour réaliser une ou plusieurs ponctions. Les contraintes du jeu de contraintes peuvent découler du type d'organe dans lequel la tumeur 200 se trouve, de la taille de la tumeur 200, de la présence de tissus nécrosés 500 au sein de la tumeur 200, ou de tout autre paramètre contraignant la réalisation des biopsies.

Dans l'exemple décrit ici, la tumeur 200 est une tumeur du poumon, et les contraintes de biopsie qui y sont associées, représentées sur la figure 3, sont les suivantes :

- les différentes biopsies doivent toutes avoir le même point d'entrée 302 dans la tumeur 200,
- les différentes trajectoires des aiguilles 304, 306, 308 sont en ligne droite et doivent être comprises dans un angle maximal de ponction 310,
- la profondeur à laquelle une aiguille de biopsie est insérée dans la tumeur est inférieure à une profondeur maximale de ponction 312.

Dans l'exemple décrit ici, l'angle maximal de ponction 310 vaut 20° et la profondeur maximale de ponction 312 vaut 2,2 cm.

Les contraintes peuvent également porter sur :

- la proximité de la zone ponctionnée par une biopsie avec les bords de la tumeur, par exemple avec des ponctions à au moins 2,5 mm desdits bords,
- l'existence de tissus nécrosés au sein de la tumeur, dans lesquels l'aiguille ne doit pas passer lors d'une biopsie,
- l'exclusion des tissus gras des zones dans lesquelles on peut ponctionner,
- la fixation d'un point d'entrée ou d'un ensemble de points d'entrée spécifique sur le bord de la tumeur,
- éviter des zones les plus vascularisées pour diminuer le risque de diffusion de cellules cancéreuses,
- la présence d'os empêchant les ponctions selon certains points d'entrée et certains angles, par exemple les côtes dans le cas d'une tumeur au poumon, et
- des spécificités de l'organe et/ou de la procédure de biopsie envisagée. Par exemple, pour la biopsie de la prostate par ultrasonographie transrectale, le patient est en position latérale gauche, ce qui limite donc l'accessibilité.

Comme il a été vu plus haut, les pixels correspondent chacun à une région de l'image en coupe de forme sensiblement carrée et de côté 2,1 mm, et la zone de ponction de l'aiguille de ponction présente un diamètre de sensiblement 0,84 mm et une longueur de 4,55 ± 1,45 mm. Ainsi, il y a deux obstacles à une analyse de l'image matricielle 14 :

1) D'une part, ses pixels sont trop gros pour représenter les hétérogénéités de la tumeur.
2) D'autre part, ses pixels sont trop petits pour que l'aiguille de biopsie prélève des tissus dans une zone de ponction avec certaines propriétés de paramètre de diffusion et d'homogénéité, incluse sensiblement dans une région correspondant à un seul pixel.

Le suréchantillonneur 20 raffine l'image matricielle afin de pallier au premier obstacle.

Pour ce faire, le suréchantillonneur 20 reçoit l'image matricielle 14 et la suréchantillonne en une image de traitement 22. L'image de traitement 22 est une image de résolution spatiale très supérieure à celle de l'image matricielle 14, comme on peut le voir en comparant la figure 2 et la figure 4. Chacun des pixels de l'image de traitement 22 correspond à une région de l'image en coupe sensiblement carrée. La résolution de l'image de traitement 22 est choisie par le suréchantillonneur 20 de telle sorte que chacun de ses pixels présente un côté de longueur inférieure au diamètre de ponction des données d'aiguille 160.

Dans le mode de réalisation décrit ici, le suréchantillonneur 20 applique une technique d'interpolation pour suréchantillonner l'image matricielle 14. Les techniques d'interpolation utilisées peuvent être l'interpolation bicubique, l'interpolation bilinéaire, le filtre de Bell, le filtre de Hermite, le filtre de Mitchell, le filtre de Lanczos, ou toute autre technique classique d'interpolation adaptée à des images. Dans l'exemple décrit ici, le suréchantillonneur 20 applique la technique d'interpolation bicubique, décrite par exemple dans l'article Keys, R. (1981). "Cubic convolution interpolation for digital image processing." IEEE transactions on Signal Processing. In Acoustics, Speech, and Signal Processing (Vol. 29, p. 1153).

En variante, le suréchantillonneur 20 peut appliquer une technique de transformée en cosinus discrète (« *Discrete Cosine Transform* » en anglais, aussi abrégé en « DCT ») pour suréchantillonner l'image matricielle 14, comme celles décrites dans l'article Dugad, R., & Ahuja, N. (2001). "A fast scheme for image size change in the compressed domain." IEEE Transactions on Circuits and Systems for Video Technology, 11(4), 461-474. et dans l'article Park, H., Park, Y., & Oh, S. K. (2003). "LIM-fold image resizing in block-DCT domain using symmetric convolution." IEEE Transactions on Image Processing, 12(9), 1016-1034. On pourrait alternativement utiliser une technique à base d'apprentissage machine, en particulier d'apprentissage profond, comme celle décrite dans l'article Wang, Z., Chen, J., & Hoi, S. C. (2019). "Deep learning for image super-resolution: A survey." arXiv preprint arXiv: 1902.06068.

L'image de traitement 22 présente des pixels dont le côté correspond à une zone sensiblement carrée de côté au moins 5 fois plus petit que le côté de la zone d'un pixel de l'image matricielle 14, de préférence 20 fois plus petit, comme dans l'exemple décrit ici.

Dans un autre mode de réalisation, le dispositif d'imagerie 1 travaille en trois dimensions, et les données d'imagerie 12 comprennent 9 images matricielles 14 en coupes successives, séparées deux à deux d'une distance de 6 mm. Les images matricielles 14 définissent ainsi des voxels de dimension 2,1 mm x 2,1 mm x 6 mm. Les images de traitement 22 définissent des voxels au moins 5 fois plus petits dans chaque dimension, de préférence 20 fois plus petits. Dans l'exemple décrit ici, les voxels des images de traitement 22 définissent chacun une zone de 0,105x0,105x0,3mm.

L'image de traitement 22 présente une résolution bien supérieure à celle de l'image matricielle 14. Les régions correspondant aux pixels de l'image de traitement sont bien plus petites que les hétérogénéités de la tumeur. Il faut maintenant regrouper les pixels de l'image de traitement 22 pour découper l'image en coupe en une pluralité de régions suffisamment homogènes pour pallier au premier obstacle et suffisamment grandes pour pallier au second obstacle.

Pour ce faire, le découpeur 30 reçoit et partitionne l'image de traitement 22 en un jeu de régions 32. Chacune des régions 34 du jeu de régions 32 comprend des pixels de l'image de traitement 22 qui sont directement voisins, au moins deux à deux.

La région 34 est associée à une valeur de paramètre de diffusion sensiblement égale à la moyenne des valeurs de paramètre de diffusion des pixels qui la composent. Cette valeur de paramètre de diffusion de la région 34 peut être par exemple choisie comme étant la valeur moyenne des valeurs de paramètre de diffusion associées aux pixels de la région 34, ou comme la valeur médiane.

Le découpeur 30 détermine le jeu de régions 32 de sorte que, pour chaque région 34, la variance des valeurs de paramètre de diffusion associés aux pixels de cette région 34 est inférieure à une valeur seuil. Les régions 34 du jeu de régions 32 sont ainsi des ensembles de pixels contigus de l'image de traitement 22 dont les valeurs de paramètre de diffusion associées sont sensiblement homogènes, ou, dit autrement, dont la variance est faible.

Le découpeur 30 détermine en outre le jeu de régions 32 de sorte que région 34 corresponde à une portion de l'image par résonance magnétique de diffusion en coupe dont les dimensions sont supérieures ou sensiblement égales aux dimensions d'une ponction réalisée au moyen d'une aiguille de biopsie des données d'aiguille 160. Ici, les portions auxquelles les régions 34 correspondent présentent des dimensions sensiblement similaires, avec par exemple des aires respectives sensiblement égales. Les paramètres de diffusion associés à chaque région 34 du jeu de régions 32 forment un jeu de paramètres de diffusion 36.

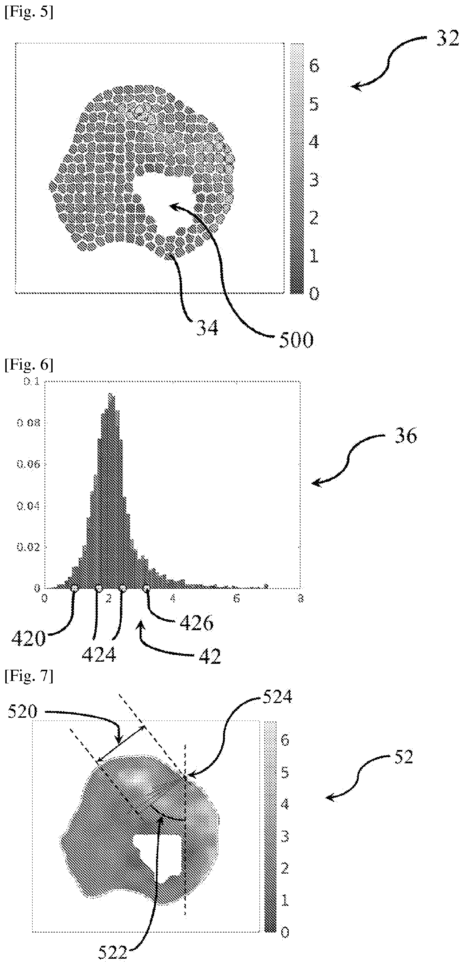

Dans un mode de réalisation, le découpeur 30 partitionne l'image de traitement 22 en le jeu de régions 32 par une méthode de superpixelisation avec pour contraintes la taille et la faible variance des valeurs de paramètre de diffusion associées aux pixels de chaque région 34. Dans l'exemple décrit ici, la méthode de superpixelisation utilisée est celle décrite dans l'article Ren, X., & Malik, J. (2003, October). "Learning a classification model for segmentation." In Proceedings Ninth IEEE International Conférence on Computer Vision, (p. 10). IEEE. Dans l'exemple décrit ici, représenté sur la figure 5, les superpixels correspondent à des portions de l'image par résonance magnétique de diffusion en coupe de taille légèrement supérieure à celle des portions correspondant aux pixels de l'image matricielle 14. En variante, on pourrait utiliser l'une des méthodes de superpixelisation décrites dans l'article Stutz, D., Hermans, A., & Leibe, B. (2018). "Superpixels: An évaluation of the state-of-the-art." Computer Vision and Image Understanding, 166, 1-27.

Dans la figure 5, une partie de l'image par résonance magnétique de diffusion en coupe n'est pas prise en compte, car il s'agit d'une région 500 correspondant à des tissus nécrosés. La détermination des données correspondant à la région 500 peut être réalisée par le suréchantillonneur 20, le découpeur 30 ou par une autre partie du dispositif d'imagerie 1. Les données correspondant à la région 500 pourraient en variante être déterminées par un opérateur, par exemple avant la mise en place de l'opération de biopsie.

En variante, le découpeur 30 peut partitionner l'image de traitement 22 en le jeu de régions 32 par une méthode de partition en polygones convexes telle que décrite dans l'article Duan, L., & Lafarge, F. (2015). "Image partitioning into convex polygons." In Proceedings of the IEEE Conférence on Computer Vision and Pattern Recognition (pp. 3119-3127). Cette méthode repose sur la construction d'un diagramme de Voronoï dont les cellules sont des polygones convexes en se conformant à des lignes droites déjà tracées au sein de l'image de traitement 22. On peut ici obtenir des régions 34 dont on peut contrôler les dimensions, et qui préservent les bords des formes ou objets au sein de l'image de traitement 22 à une échelle subpixelique. Le jeu de régions 32 fournit ici une représentation des hétérogénéités au sein de l'image de traitement 22.

Dans le cas tridimensionnel, où plusieurs images de traitement 22 sont produites par le suréchantillonneur 20, le découpeur 30 partitionne les images de traitement 22 à l'aide d'une méthode de supervoxelisation, comme l'une de celles décrites dans l'article Xu, C., & Corso, J. J. (2012, June). "Evaluation of super-voxel methods for early video processing." In 2012 IEEE conférence on computer vision and pattern récognition (pp. 1202-1209). IEEE.

Les régions 34 déterminées par le découpeur 30 sont ainsi

- suffisamment grandes pour que la zone de ponction d'une aiguille puisse être sensiblement incluse dans une des régions 34, et
- suffisamment homogènes pour qu'un prélèvement au sein d'une région 34 soit représentatif de cette région 34.

Le sélecteur 40 détermine, parmi les régions 34, lesquelles sont pertinentes pour un prélèvement, de sorte que les valeurs de paramètre de diffusion dans ces régions soient représentatives de l'ensemble des paramètres de diffusion.

Pour ce faire, le sélecteur 40 détermine au sein du jeu de paramètres de diffusion 36 un sous-jeu de paramètres de diffusion 42 qui comprend autant de paramètres de diffusion que le nombre de ponctions à effectuer des données de procédure 162. Le nombre de ponctions à effectuer étant au moins égal à deux, le sous-jeu de paramètres de diffusion 42 comprend au moins deux paramètres de diffusion. Un premier paramètre de diffusion 420 des paramètres de diffusion est choisi dans le premier décile des valeurs de paramètre de diffusion du jeu de paramètres de diffusion 36. Un deuxième paramètre de diffusion 426 des paramètres de diffusion est choisi dans le dernier décile des valeurs de paramètre de diffusion du jeu de paramètres de diffusion 36. Le premier paramètre de diffusion 420 et le deuxième paramètre de diffusion 426 peuvent par exemple chacun valoir la valeur médiane de leur décile respectif. Cela permet de s'assurer que le sous-jeu de paramètres de diffusion 42 déterminé par le sélecteur 40 est représentatif des valeurs de paramètre de diffusion des régions 34 du jeu de régions 32, en particulier des valeurs extrémales des paramètres de diffusion du jeu de paramètres de diffusion 36.

Dans un mode de réalisation, le premier paramètre de diffusion 420 et le deuxième paramètre de diffusion 426 sont choisis respectivement dans le 5-ième et le 95-ième percentile du jeu de paramètres de diffusion, de préférence dans le 2-ième et le 98-ième percentile. Ici, le premier paramètre de diffusion 420 et le deuxième paramètre de diffusion 426 valent chacun la valeur médiane de leur quantile respectif.

Lorsque le nombre de ponctions à effectuer des données de procédure 162 est supérieur à 2, le sélecteur 40 détermine des paramètres de diffusion 424 supplémentaires en plus du premier paramètre de diffusion 420 et du deuxième paramètre de diffusion 426. Les valeurs respectives de ces paramètres de diffusion 424 supplémentaires sont comprises entre la valeur du premier paramètre de diffusion 420 et la valeur du deuxième paramètre de diffusion 426.

Dans un mode de réalisation, le sélecteur 40 choisi les paramètres de diffusion 424 compris entre le premier paramètre de diffusion 420 et le deuxième paramètre de diffusion 426 de sorte que les paramètres de diffusion du sous-jeu de paramètres de diffusion 42 présentent des valeurs équidistantes deux à deux. Pour ce faire, le sélecteur 40 applique l'équation (1) de l'Annexe A, où Dmin est la valeur du premier paramètre de diffusion 420, Dmax est la valeur du deuxième paramètre de diffusion 426, N est le nombre de ponctions à effectuer dans une procédure de biopsie des données de procédure 162, j est un nombre entier valant entre 1 et le nombre de ponctions à effectuer inclus et Dj est la valeur du j-ième paramètre de diffusion du sous-jeu de paramètres de diffusion 42. Le sous-jeu de paramètres de diffusion 42 ainsi obtenu est représenté sur la figure 6, où l'axe des abscisses indique les valeurs de paramètre de diffusion et l'axe des ordonnées indique la probabilité d'avoir une valeur de paramètre de diffusion. Le sous-jeu de paramètres de diffusion 42 comprend peu de paramètres de diffusion (4, dans l'exemple décrit ici), mais ces derniers sont très représentatifs de l'ensemble des paramètres de diffusion du jeu de paramètres de diffusion 36, y compris pour les valeurs extrêmes.

De manière alternative, lorsque le jeu de paramètres de diffusion 36 présente une distribution de ses paramètres de diffusion autour de la valeur médiane M des paramètres de diffusion du jeu de paramètres de diffusion 36 très asymétrique, le sélecteur 40 détermine les paramètres de diffusion 424 selon la formule (2) de l'Annexe A, où Dini est déterminé selon la formule (3) de l'Annexe A et Delta est déterminé selon la formule (4) de l'Annexe A. Cette variante permet de donner moins de poids à la plus longue queue de la distribution des paramètres de diffusion du jeu de paramètres de diffusion 36, de sorte que le sous-jeu de paramètres de diffusion 42 est représentatif de la répartition des paramètres de diffusion du jeu de paramètres de diffusion 36. Cette asymétrie peut par exemple apparaître en cas de bruit trop important sur l'image matricielle 12, ce qui perturbe davantage les petites valeurs de paramètre de diffusion que les hautes valeurs de paramètre de diffusion. La détermination de cette asymétrie, et donc le choix de cette méthode asymétrique ou de la méthode par équidistance décrite plus haut, peut être réalisée manuellement, ou automatiquement par le sélecteur 40.

Dans un mode de réalisation, le sélecteur 40 calcule le coefficient d'asymétrie (« *skewness* » en anglais) du jeu de paramètres de diffusion 36. Si ce coefficient d'asymétrie dépasse un certain seuil, le sélecteur 40 détermine les paramètres de diffusion 424 selon la formule (2). Dans les autres cas, le sélecteur 40 applique la formule (1).

Le sélecteur 40 renvoie un sous-jeu de régions 44 comprenant des régions 46 tirées du jeu de régions 32. Chaque région 46 du sous-jeu de régions 44 est associée à un paramètre de diffusion sensiblement égal à l'un des paramètres de diffusion du sous-jeu de paramètres de diffusion 42. En outre, dans l'exemple décrit ici, tous les paramètres de diffusion sont chacun associé à au moins une des régions 46. En variante, pour chaque paramètre de diffusion du sous-jeu de paramètres de diffusion 42, le sous-jeu de régions 44 peut comprendre toutes les régions 34 du jeu de régions 32 associées audit paramètre de diffusion du sous-jeu de paramètres de diffusion 42. Dans le mode de réalisation où le jeu de paramètres de diffusion est découpé en quantiles, le paramètre de diffusion de chaque région 46 du sous-jeu de régions 44 est inclus dans le même quantile que le paramètre de diffusion du sous-jeu de paramètres de diffusion 42 associé.

Le sous-jeu de régions 44 comprend des régions 46 présentant des valeurs de paramètre de diffusion bien réparties dans le jeu de paramètres de diffusion 36. De fait, un prélèvement dans plusieurs de ces régions 46 dont les valeurs de paramètre de diffusion sont deux à deux différentes est représentatif de l'ensemble des régions 34.

Cependant, la tumeur 200 présente un certain nombre de particularités comme la forme du jeu de contraintes 300 ou la présence de régions nécrotiques 500. Sur la base de ces particularités, le guide 50 détermine parmi le sous-jeu de régions 44 quelles sont les régions à prélever, et comment réaliser ces prélèvements.

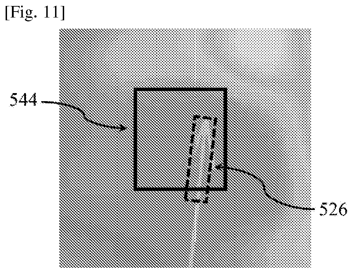

Pour ce faire, le guide 50 détermine, à partir du sous-jeu de régions 44, des données de jeux de paramètres de ponction 52, dont le nombre est égal au nombre de ponctions à effectuer des données de procédure 162. Chaque jeu de paramètres de ponction des données de jeux de paramètres de ponction 52 comprend une profondeur de ponction 520, une orientation de ponction 522 et un point d'entrée de ponction 524, représentés dans l'exemple décrit ici sur la figure 7. Chaque jeu de paramètres de ponction définit avec les données d'aiguille 160 une zone de ponction 526, visible figure 11, qui est sensiblement incluse dans l'une des régions 46 du sous-jeu de régions 44. Le guide 50 détermine les jeux de paramètres de ponction des données de jeux de paramètres de ponction 52 de sorte que les jeux de paramètres de ponction respectent ensemble le jeu de contraintes 300 des données de procédure 162.

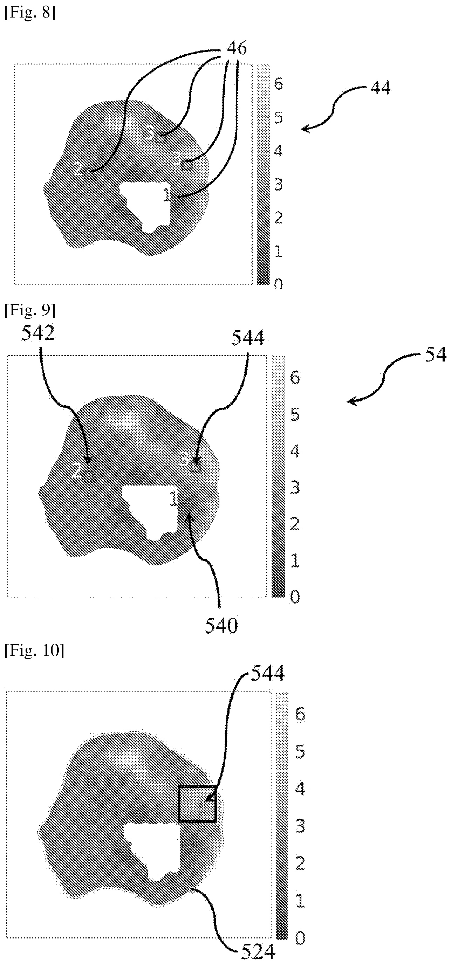

Dans l'exemple représenté sur la figure 8, le guide 50 reçoit un sous-jeu de régions 44 issu du sélecteur 40 comprenant quatre régions 46. Ici, le sous-jeu de paramètres de diffusion 42 comprend trois paramètres de diffusion P1, P2 et P3. Les paramètres de diffusion P1 et P2 sont chacun associés à une unique région du sous-jeu de régions 44 respectivement référencées « 1 » et « 2 » sur la figure 8. Le paramètre P3 est associé à deux régions du sous-jeu de régions 44 référencées « 3 » sur la figure 8. Le guide 50 sélectionne alors, parmi ces régions 46, un jeu de régions 54, représenté sur la figure 9, adapté pour une ponction en fonction du jeu de contraintes 300. Comme la procédure prévoit la réalisation de trois biopsies, ce jeu de régions 54 comporte trois régions 540, 542 et 544. Les régions 540 et 542 correspondent respectivement aux paramètres P1 et P2. La région 544 étiquetée « 3 » est sélectionnée parmi l'une des deux régions correspondant au paramètre de diffusion P3 sur la figure 8.

Dans certains cas, le guide 50 peut déterminer que le sous-jeu de régions 44 est incompatible avec le jeu de contraintes 300. Cela signifie qu'il n'est pas possible d'effectuer autant de ponctions que prévu initialement en respectant le jeu de contraintes 300. Dans ce cas, le sélecteur 40 est exécuté pour déterminer un nouveau sous-jeu de paramètres de diffusion 42 comprenant un paramètre de diffusion en moins que précédemment, puis un sous-jeu de régions 44. Cela est répété jusqu'à ce que le guide 50 détermine un sous-jeu de régions 44 compatible avec le jeu de contraintes 300, ou qu'il soit déterminé que la procédure de biopsie est impossible.

Dans un autre exemple, représenté sur la figure 10 et la figure 11, le nouveau jeu de régions 54 sélectionné par le guide 50 comporte trois régions 540, 542 et 544. La zone de ponction 526 des données de jeux de paramètres de ponction 52 déterminées par le guide 50 est ici sensiblement incluse dans la région 544 du nouveau jeu de régions 54.

Les données de jeux de paramètres de ponction 52 déterminées par le guide 50 sont fournies à un chirurgien ou un radiologue qui est alors en mesure de réaliser des biopsies bien plus pertinentes que s'il les choisissait à la main ou aléatoirement. En outre, comme le nombre de biopsies réalisé est faible, inférieur à 4 dans l'exemple décrit ici, les risques médicaux sont fortement diminués.

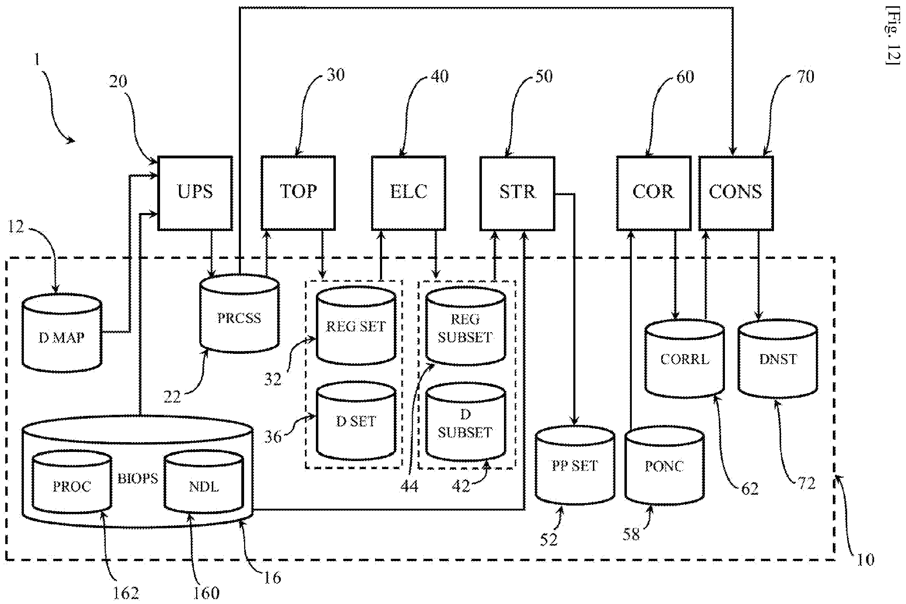

Il est maintenant fait référence aux figures 12 à 15.

Dans le mode de réalisation décrit ici, le dispositif d'imagerie 1 récupère le résultat d'une procédure de biopsie réalisée conformément aux données de jeux de paramètres de ponction 52. Ce résultat est stocké dans la mémoire sous la forme de données de ponction 58. A partir des données de ponction 58, le dispositif d'imagerie 1 reconstruit une carte de densité de la tumeur 200.

Pour ce faire, le dispositif d'imagerie 1 comprend un corrélateur 60 et un constructeur 70. Le corrélateur 60 tire des données de ponction 58 une corrélation entre les valeurs de paramètre de diffusion du jeu de paramètres de diffusion 36 et des valeurs de densité cellulaire dans la tumeur 200. La densité cellulaire peut être une densité de cellule totale ou une densité de cellules cancéreuses. Le constructeur 70 utilise cette corrélation pour reconstruire une carte de densité cellulaire au sein de la tumeur 200.

Les données de ponction 58 comprennent un jeu de densités cellulaires. Chaque densité cellulaire est associée à l'une des zones de ponction 526 des données de jeux de paramètres de ponction 52. Chaque densité présente une valeur représentant une densité cellulaire de la zone de ponction 526 qui lui est associée.

Ces données de ponction 58 peuvent être obtenues après une procédure de biopsie par un chirurgien ou radiologue et après analyse des échantillons prélevés dans les zones de ponction 526 des données de jeux de paramètres de ponction 52. L'analyse de ces données de ponction 58 peut être réalisée par une méthode d'histologie comme celle décrite dans l'article Yin, Y., Sedlaczek, O., Müller, B., Warth, A., Gonzâlez-Vallinas, M., Lahrmann, B., Grabe, N., Kauczor, HU., Breuhahn, K., Vignon-Clementel, IE., Drasdo, D. (2018). "Tumor cell load and heterogeneity estimation from diffusion-weighted MRI calibrated with histological data: an example from lung cancer." IEEE transactions on medical imaging, 37(1), 35-46.

Le corrélateur 60 reçoit les données de ponction 58 et associe à chaque valeur de densité cellulaire associée à l'une des zones de ponction 526 la valeur de paramètre de diffusion de la région 46 dans laquelle est sensiblement incluse ladite zone de ponction 526. Le corrélateur 60 détermine des données de corrélation 62 qui associent des valeurs de densité cellulaire à des valeurs de paramètre de diffusion parmi le jeu de paramètres de diffusion 36.

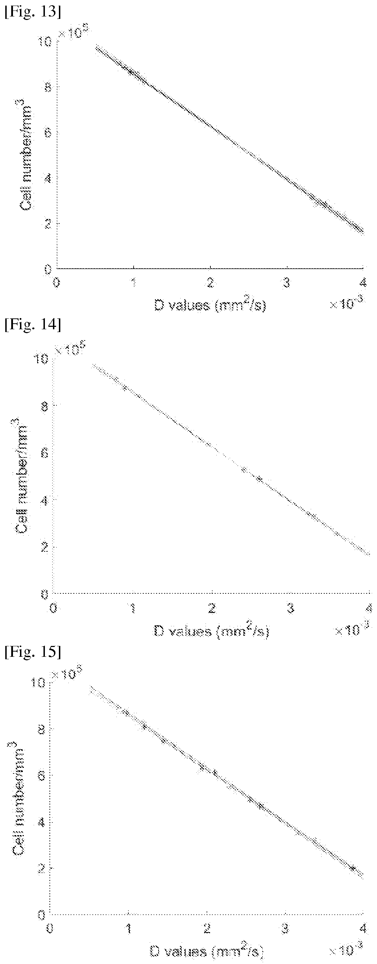

Dans le mode de réalisation décrit ici, le corrélateur 60 détermine des paires associant chacune une valeur de paramètre de diffusion et une valeur de densité cellulaire associées à une des zones de ponction des données de jeux de paramètres de ponction 52. Sur la base de ces paires, le corrélateur 60 réalise une interpolation linéaire dont le résultat permet de relier les valeurs de paramètre de diffusion du jeu de paramètres de diffusion 36 à des valeurs de densité cellulaire. Le corrélateur 60 détermine des données de corrélation 62 sur la base du résultat de l'interpolation linéaire. Les figures 13, 14 et 15 représentent des interpolations linéaires pour un nombre de ponctions des données de procédure 162 valant respectivement deux, trois et quatre. Les figures 13, 14 et 15 sont munies d'un axe des abscisses indiquant les valeurs de paramètre de diffusion, et un axe des ordonnées indiquant une densité cellulaire. Plus la valeur de paramètre de diffusion est élevée, ce qui indique une grande mobilité des molécules d'eau au sein des tissus, plus la densité cellulaire est faible.

En variante, l'interpolation réalisée par le corrélateur 60 peut être une interpolation polynomiale strictement monotone, ou autre fonction strictement monotone.

Le constructeur 70 détermine des données de densité 72 à partir de l'image de traitement 22 et des données de corrélations 62. Les données de densité 72 comprennent une image de densité. Chaque pixel de l'image de densité correspond à une région de l'image en coupe à laquelle correspond également un pixel de l'image de traitement 22. Le constructeur 70 associe à chaque pixel de l'image de densité la valeur de densité cellulaire associée dans les données de corrélation 62 à la valeur de paramètre de diffusion dudit pixel de l'image de traitement 22.

Ainsi, à partir de l'image matricielle 14, le dispositif d'imagerie 1 détermine ici un nombre réduit de prélèvements à réaliser, puis, partant du résultat de ces prélèvements, est en mesure de reconstruire une carte précise de la densité cellulaire de la tumeur 200.

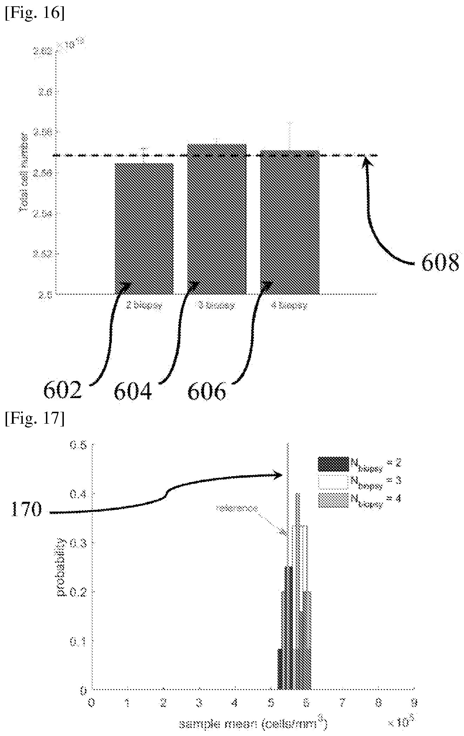

Il est fait référence à la figure 16. La figure 16 est munie d'un axe des abscisses indiquant un nombre de ponctions effectuées, et d'un axe des ordonnées indiquant une valeur de charge tumorale.

Dans un mode de réalisation, non représenté sur les figures, le dispositif d'imagerie 1 comprend en outre un estimateur agencé pour déterminer une charge tumorale de la tumeur 200. L'estimateur réalise ici une intégration numérique surfacique sur toute l'aire de l'image de densité de laquelle il tire la charge tumorale. La figure 16 représente une charge tumorale 602, 604, 606 déterminée par l'estimateur, selon que le nombre de ponctions soit de deux, trois ou quatre, respectivement, et compare cette charge tumorale à une charge tumorale de référence 608. Plus le nombre de ponctions est élevé, et plus la charge tumorale déterminée par l'estimateur est proche de la charge tumorale de référence 608. Le dispositif d'imagerie 1 est ainsi capable de mesurer une charge tumorale avec seulement deux biopsies, là où les méthodes classiques avec des biopsies prises aléatoirement avec 8, voire 10 biopsies sont moins précises. Avec trois ou quatre biopsies, la charge tumorale mesurée est encore plus précise, tout en conservant un nombre de ponctions contenu.

Ici, le dispositif d'imagerie 1 est ainsi capable de déterminer la charge tumorale de la tumeur 200 à partir du résultat de prélèvements suivant les données de jeux de paramètres de ponction 52, et de visualiser et/ou quantifier l'hétérogénéité de la tumeur.

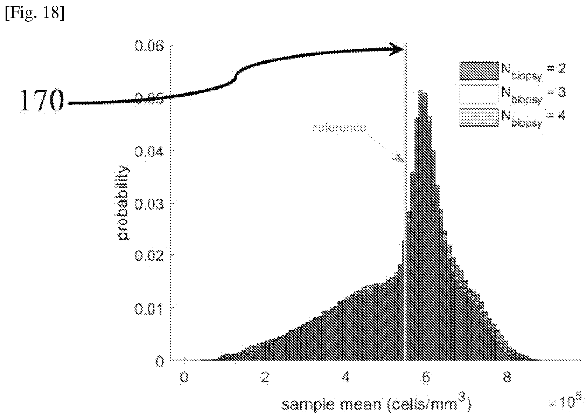

Il est fait référence aux figures 17 et 18. Chaque figure représente trois histogrammes basés sur la détermination de charges tumorales à partir de deux, trois et quatre biopsies. Plus précisément, l'abscisse indique une valeur de charge tumorale, et l'ordonnée indique le pourcentage de biopsies présentant une valeur de charges tumorales estimées par rapport à l'ensemble des biopsies réalisées. Ainsi, sur la figure 17, 10% des procédures de biopsies impliquant 2 biopsies induisent une charge tumorale estimée de 600 000 cellules par mm$^3$. Sur ces figures, une valeur de charge tumorale de référence 170 est également représentée afin d'indiquer la valeur cible idéale qui devrait être estimée.

Afin de montrer l'intérêt de l'invention par rapport à l'existant, on a représenté sur la figure 17 un histogramme tiré de biopsies choisies au moyen du dispositif selon l'invention, tandis que la figure 18 représente un histogramme réalisé à partir de ponctions réalisées de manière aléatoire en respectant le jeu de contraintes 300.

Ces figures montrent que les données de jeux de paramètres de ponction 52 déterminées par le dispositif d'imagerie 1 permettent de réaliser des ponctions permettant de déterminer une charge tumorale bien plus proche de la charge tumorale réelle que des ponctions aléatoires. Ces figures montrent également que les mesures de charge tumorale réalisées sur la base des données de jeux de paramètre de ponction 52 présentent une dispersion bien moindre que les mesures réalisées avec des ponctions aléatoires.

L'utilisation de données de jeux de paramètres de ponction 52 déterminées par le dispositif d'imagerie 1 permet donc au corrélateur 60, au constructeur 70 et à l'estimateur de déterminer une charge tumorale beaucoup plus fiable, c'est-à-dire proche de la valeur de référence 170.

Le dispositif d'imagerie 1 permet à un chirurgien ou un radiologue de mesurer précisément et de manière robuste une charge tumorale à partir d'un très faible nombre de ponctions, en général trois à cinq fois moins que les procédures classiques.

Le corrélateur 60, le constructeur 70 et l'estimateur sont ici des programmes exécutés par le processeur de l'ordinateur. Les variantes décrites pour le suréchantillonneur 20, le découpeur 30, le sélecteur 40 et le guide 50 peuvent également s'appliquer au corrélateur 60, au constructeur 70 et à l'estimateur.

Dans ce qui précède, le découpeur 30 détermine des régions 34 présentant une taille supérieure ou sensiblement égale à la zone de ponction d'une aiguille de biopsie. En variante, le découpeur 30 pourrait déterminer des régions 34 dont les dimensions sont inférieures à la longueur de ponction, mais supérieures au diamètre de ponction. Pour les biopsies réalisées sur cette base, les mesures histologiques ne seraient alors réalisées que sur la partie des prélèvements correspondant aux régions 540, 542, 544.

Le dispositif peut aussi en variante recevoir dans la mémoire 10 des données d'autres types d'imagerie non-invasive dont les pixels des images matricielles 14 sont associés, au lieu du paramètre de diffusion dans le cas de DWI, à un paramètre qui reflète la densité cellulaire.

Annexe A

$$(1) \quad Dj = Dmin + (j-1)(Dmax - Dmin)/(N-1)$$

$$(2) \quad Dj = Dini + Delta(j-1)$$

$$(3) \quad Dini = M - min(|M - Dmin|, |M - Dmax|)$$

$$(4) \quad Delta = 2(M - Dini)/(N-1)$$

**Revendications**

**1.** Dispositif d'imagerie comprenant :

- une mémoire (10), agencée pour recevoir

des données d'imagerie (12) comprenant au moins une image matricielle (14) tirée d'une image par résonnance magnétique de diffusion en coupe d'une tumeur, chaque pixel de ladite image matricielle (14) étant associé à un paramètre de diffusion dont la valeur représente la mobilité de molécules d'eau au sein de ladite tumeur,
des données de biopsie (16) comprenant des données d'aiguille (160) définissant les dimensions d'une ponction réalisée au moyen d'une aiguille de biopsie, et
des données de procédure (162) comprenant un nombre de ponctions à effectuer dans une procédure de biopsie supérieur ou égal à 2 et un jeu de contraintes (300) associées à des contraintes interventionnelles à respecter pour réaliser le nombre de ponctions à effectuer,

- un suréchantillonneur (20), agencé pour suréchantillonner l'image matricielle (14) en une image de traitement (22) dont la résolution spatiale est telle que chaque pixel de l'image de traitement (22) correspond à une région de l'image en coupe sensiblement carrée dont le côté présente une longueur inférieure à une dimension de diamètre d'aiguille des données d'aiguille (160),
- un découpeur (30) agencé pour partitionner l'image de traitement (22) issue du suréchantillonneur (20) en un jeu de régions (32) dans lequel chaque région (34) comprend des pixels de l'image de traitement (22) directement voisins au moins deux à deux, est associée à un paramètre de diffusion dont la valeur est sensiblement égale à la moyenne des valeurs des paramètres de diffusion associés aux pixels qui la composent, est telle que la variance des valeurs des paramètres de diffusion associés aux pixels de la région est inférieure à une valeur de variance donnée, correspond à une portion de l'image par résonnance magnétique de diffusion en coupe de ladite tumeur dont les dimensions sont supérieures ou sensiblement égales aux dimensions d'une ponction réalisée au moyen d'une aiguille de biopsie des données de biopsie (16), et dans lequel les paramètres de diffusion associés à chaque région (34) du jeu de régions (32) forment un jeu de paramètres de diffusion (36),
- un sélecteur (40), agencé pour

déterminer au sein du jeu de paramètres de diffusion (36) un sous-jeu de paramètres de diffusion (42) comprenant autant de paramètres de diffusion que le nombre de ponctions à effectuer des données de procédure (162), le sous-jeu de paramètres de diffusion (42) comprenant au moins un premier paramètre de diffusion (420) choisi dans le premier décile des valeurs de paramètre de diffusion du jeu de paramètres de diffusion (36), un deuxième paramètre de diffusion (426) dans le dernier décile des valeurs de paramètre de diffusion du jeu de paramètres de diffusion (36), et, lorsque le nombre de ponctions à effectuer des données de procédure (162) est supérieur à 2, des paramètres de diffusion (424) dont les valeurs de paramètre de diffusion respectives sont comprises entre la valeur du premier paramètre de diffusion (420) et la valeur du deuxième paramètre de diffusion (426),
renvoyer un sous-jeu de régions (44) tirées du jeu de régions (32) et dans lequel chaque région (46) est associée à un paramètre de diffusion sensiblement égal à l'un des paramètres du sous-jeu de paramètres de diffusion (42),

- un guide (50), agencé pour déterminer, à partir du sous-jeu de régions (44), des données de jeux de paramètres de ponction (52) dont le nombre est égal au nombre de ponctions à effectuer des données de procédure (162), chaque jeu de paramètres de ponction comprenant une profondeur de ponction (520), une orientation de ponction (522) et un point d'entrée de ponction (524) et définissant avec les données d'aiguille (160) une zone de ponction (526) qui est sensiblement incluse dans une des régions (544) du sous-jeu de régions (54), de telle sorte que les jeux de paramètres de ponction (52) respectent ensemble le jeu de contraintes (300) des données de procédure (162).

2. Dispositif d'imagerie selon la revendication 1, dans lequel le suréchantillonneur (20) est agencé pour suréchantillonner l'image matricielle (14) en l'image de traitement (22) en réalisant une interpolation.

3. Dispositif d'imagerie selon la revendication 2, dans lequel le suréchantillonneur (20) est agencé pour réaliser une interpolation bicubique pour suréchantillonner l'image matricielle (14) en l'image de traitement (22).

4. Dispositif d'imagerie selon l'une des revendications précédentes, dans lequel le découpeur (30) est agencé pour partitionner l'image de traitement (22) issue du suréchantillonneur (20) par une méthode de superpixelisation.

5. Dispositif d'imagerie selon l'une des revendications précédentes, dans lequel le nombre de ponctions à effectuer dans une procédure de biopsie des données de procédure (162) que stocke la mémoire (10) est inférieur ou égal à 4.

6. Dispositif d'imagerie selon l'une des revendications précédentes, dans lequel le sélecteur (40) choisit le premier paramètre de diffusion (420) dans le deuxième percentile des valeurs de paramètre de diffusion du jeu de paramètres de diffusion (36), et le deuxième paramètre de diffusion (426) dans le quatre-vingt-dix-huitième percentile des valeurs de paramètre de diffusion du jeu de paramètres de diffusion (36).

7. Dispositif d'imagerie selon l'une des revendications précédentes, dans lequel le premier paramètre de diffusion (420) et le deuxième paramètre de diffusion (426) sont chacun égaux à la valeur médiane de leur quantile respectif.

8. Dispositif d'imagerie selon l'une des revendications précédentes, dans lequel lorsque le nombre de ponctions à effectuer des données de procédure (162) est supérieur à 2, le sélecteur (40) choisit les paramètres de diffusion (424) du sous-jeu de paramètres de diffusion (42) de sorte qu'ils soient équidistants deux à deux.

9. Dispositif d'imagerie selon l'une des revendications 1 à 7, dans lequel lorsque le nombre de ponctions ($N$) à effectuer des données de procédure (162) est supérieur à 2, le sélecteur (40) détermine une valeur médiane (M) des paramètres de diffusion du jeu de paramètres de diffusion (36), et détermine les paramètres de diffusion ($Dj$), j étant un entier naturel compris entre 1 et le nombre de ponctions (N) à effectuer des données de procédure (162), du sous-jeu de paramètres de diffusion (42) selon l'équation :

$$Dj = Dini + Delta(j - 1),$$

où *Dini* vérifie l'équation :

$$Dini = M - min(|M - Dmin|, |M - Dmax|)$$

et *Delta* vérifie l'équation :

$$Delta = 2(M - Dini)/(N-1),$$

*Dmin* étant égal à la valeur du premier paramètre de diffusion (420) déterminé par le sélecteur (40) et *Dmax* étant égal à la valeur du deuxième paramètre de diffusion (426) du sous-jeu de paramètres de diffusion (42) déterminé par le sélecteur (40).

10. Dispositif d'imagerie selon l'une des revendications précédentes, dans lequel la profondeur de ponction (520), l'orientation de ponction (522), le point d'entrée de ponction (524) de chacun des jeux de paramètres de ponction (52) déterminés par le guide (50) définissent avec les données de biopsie (16) une trajectoire de ponction de l'aiguille de biopsie, et dans lequel le jeu de contraintes (300) des données de procédure (162) stockées dans la mémoire (10) comprend une ou plusieurs contraintes choisies dans le groupe comprenant :

   - les ponctions d'une procédure de biopsie doivent avoir le même point d'entrée (524) dans la tumeur (200),
   - les trajectoires de ponction sont en ligne droite et doivent être comprises dans un angle maximal de ponction,
   - la profondeur de ponction (520) est inférieure à une profondeur maximale de ponction,
   - la zone de ponction (526) est éloignée des bords de la tumeur (200) d'une distance prédéterminée,
   - si la tumeur (200) comprend une zone nécrosée (500), la trajectoire de ponction ne pénètre pas ladite zone nécrosée (500),
   - si la tumeur (200) comprend une zone de tissus gras, aucune des zones de ponction (526) n'intersecte ladite zone de tissus gras,
   - au moins un des points d'entrée (524) des données de jeux de paramètres de ponction (52) est fixé à l'avance.

11. Dispositif d'imagerie selon l'une des revendications précédentes, dans lequel la mémoire (10) est en outre agencée pour recevoir des données de ponction (58) comprenant un jeu de densités cellulaires, chaque densité cellulaire étant associée à l'une des zones de ponction (526) des données de jeux de paramètres de ponction (52), chaque densité cellulaire ayant une valeur représentant une densité cellulaire de ladite zone de ponction (526), le dispositif d'imagerie (1) comprenant en outre :

   - un corrélateur (60), agencé pour associer à chaque valeur de densité cellulaire associée à l'une des zones de ponction (526) la valeur de paramètre de diffusion de la région (544) dans laquelle est sensiblement incluse ladite zone de ponction (526), et ce pour le jeu de région (54), et pour en tirer des données de corrélation (62) associant des valeurs de densité cellulaire et des valeurs de paramètre de diffusion parmi le jeu de paramètres de diffusion (36), et
   - un constructeur (70), agencé pour déterminer des données de densité (72) à partir de l'image de traitement (22) et des données de corrélations (62), les données de densité (72) comprenant une image de densité dont chaque pixel, qui correspond à une région de l'image en coupe, est associé à une densité cellulaire dont la valeur est associée dans les données de corrélation (62) à la valeur de paramètre de diffusion d'un pixel de l'image de traitement (22) correspondant à ladite région de l'image en coupe dudit pixel.

12. Dispositif d'imagerie selon la revendication 11, dans lequel le corrélateur (60) réalise une interpolation linéaire ou non linéaire strictement monotone sur la base de paires formées d'une valeur de paramètre de diffusion et d'une valeur de densité cellulaire associées à une zone de ponction (526) et tire du résultat de ladite interpolation les données de corrélation (62).

13. Dispositif d'imagerie selon la revendication 11 ou 12, dans lequel la densité cellulaire des données de ponction et des données de densité (72) correspond à une densité de cellules totale ou une densité de cellules cancéreuses.

14. Dispositif d'imagerie selon l'une des revendications 11 à 13 comprenant en outre un estimateur agencé pour déterminer une charge tumorale de la tumeur (200) sur la base des données de densité (72).

**Patentansprüche**

1. Bildgebungsvorrichtung, die enthält:

- einen Speicher (10), der eingerichtet ist, zu empfangen

Bildgebungsdaten (12), die mindestens ein Matrixbild (14) enthalten, das von einem Diffusionsmagnetresonanz-Schnittbild eines Tumors gewonnen wird, wobei jedes Pixel des Matrixbilds (14) einem Diffusionsparameter zugeordnet ist, dessen Wert die Mobilität von Wassermolekülen innerhalb des Tumors darstellt, Biopsiedaten (16), die Nadeldaten (160) enthalten, die die Abmessungen einer Punktion definieren, die mittels einer Biopsienadel hergestellt wird, und
Prozedurdaten (162), die eine Anzahl von in einer Biopsieprozedur auszuführenden Punktionen größer als oder gleich 2 und einen Satz von Voraussetzungen (300) enthalten, die interventionellen Voraussetzungen zugeordnet sind, die zu beachten sind, um die Anzahl von auszuführenden Punktionen zu realisieren,

- einen Überabtaster (20), der eingerichtet ist, um das Matrixbild (14) in ein Verarbeitungsbild (22) überabzutasten, dessen räumliche Auflösung so ist, dass jedes Pixel des Verarbeitungsbilds (22) einer im Wesentlichen quadratischen Region des Schnittbilds entspricht, deren Seite eine geringere Länge als eine Nadeldurchmesserabmessung der Nadeldaten (160) aufweist,
- einen Zuschneider (30), der eingerichtet ist, das vom Überabtaster (20) stammende Verarbeitungsbild (22) in einen Satz von Regionen (32) zu partitionieren, wobei jede Region (34)

Pixel des Verarbeitungsbilds (22) enthält, die mindestens paarweise direkt benachbart sind,
einem Diffusionsparameter zugeordnet ist, dessen Wert im Wesentlichen gleich dem Mittelwert der Werte der Diffusionsparameter ist, die den Pixeln zugeordnet sind, die sie bilden,
so ist, dass die Varianz der Werte der den Pixeln der Region zugeordneten Diffusionsparameter geringer ist als ein gegebener Varianzwert,
einem Teil des Diffusionsmagnetresonanz-Schnittbilds des Tumors entspricht, dessen Abmessungen größer als die oder gleich den Abmessungen einer Punktion sind, die mittels einer Biopsienadel der Biopsiedaten (16) realisiert wird,
und wobei die jeder Region (34) des Satzes von Regionen (32) zugeordneten Diffusionsparameter einen Satz von Diffusionsparametern (36) bilden,

- einen Selektor (40), der eingerichtet ist, um

innerhalb des Satzes von Diffusionsparametern (36) einen Teilsatz von Diffusionsparametern (42) zu bestimmen, der so viele Diffusionsparameter wie die Anzahl von auszuführenden Punktionen der Prozedurdaten (162) enthält, wobei der Teilsatz von Diffusionsparametern (42) mindestens einen ersten Diffusionsparameter (420), der im ersten Dezil der Diffusionsparameterwerte des Satzes von Diffusionsparametern (36) ausgewählt wird, einen zweiten Diffusionsparameter (426) im letzten Dezil der Diffusionsparameterwerte des Satzes von Diffusionsparametern (36), und, wenn die Anzahl von auszuführenden Punktionen der Prozedurdaten (162) höher ist als 2, Diffusionsparameter (424) enthält, deren jeweilige Diffusionsparameterwerte zwischen dem Wert des ersten Diffusionsparameters (420) und dem Wert des zweiten Diffusionsparameters (426) enthalten sind,
einen Teilsatz von Regionen (44) zurückzuschicken, die aus dem Satz von Regionen (32) gewonnen werden, und in dem jede Region (46) einem Diffusionsparameter im Wesentlichen gleich einem der Parameter des Teilsatzes von Diffusionsparametern (42) zugeordnet ist,

- eine Führung (50), die eingerichtet ist, um ausgehend vom Teilsatz von Regionen (44) Daten von Sätzen von Punktionsparametern (52) zu bestimmen, deren Anzahl gleich der Anzahl von auszuführenden Punktionen der Prozedurdaten (162) ist, wobei jeder Satz von Punktionsparametern eine Punktionstiefe (520), eine Punktionsausrichtung (522) und einen Punktionseintrittspunkt (524) enthält und mit den Nadeldaten (160) einen Punktionsbereich (526) definiert, der im Wesentlichen in einer der Regionen (544) des Teilsatzes von Regionen (54) enthalten ist, so dass die Sätze von Punktionsparametern (52) zusammen den Satz von Voraussetzungen (300) der Prozedurdaten (162) beachten.

2. Bildgebungsvorrichtung nach Anspruch 1, wobei der Überabtaster (20) eingerichtet ist, um das Matrixbild (14) in ein Verarbeitungsbild (22) überabzutasten, indem eine Interpolation realisiert wird.

3. Bildgebungsvorrichtung nach Anspruch 2, wobei der Überabtaster (20) eingerichtet ist, um eine bikubische Interpolation zu realisieren, um das Matrixbild (14) in das Verarbeitungsbild (22) überabzutasten.

4. Bildgebungsvorrichtung nach einem der vorhergehenden Ansprüche, wobei der Zuschneider (30) eingerichtet ist, um das vom Überabtaster (20) stammende Verarbeitungsbild (22) durch eine Superpixelisierungsmethode zu partitionieren.

5. Bildgebungsvorrichtung nach einem der vorhergehenden Ansprüche, wobei die Anzahl von auszuführenden Punktionen in einer Biopsieprozedur der Prozedurdaten (162), die der Speicher (10) speichert, niedriger als oder gleich 4 ist.

6. Bildgebungsvorrichtung nach einem der vorhergehenden Ansprüche, wobei der Selektor (40) den ersten Diffusionsparameter (420) im zweiten Perzentil der Diffusionsparameterwerte des Satzes von Diffusionsparametern (36) und den zweiten Diffusionsparameter (426) im achtundneunzigsten Perzentil der Diffusionsparameterwerte des Satzes von Diffusionsparametern (36) wählt.

7. Bildgebungsvorrichtung nach einem der vorhergehenden Ansprüche, wobei der erste Diffusionsparameter (420) und der zweite Diffusionsparameter (426) je gleich dem Medianwert ihres jeweiligen Quantils sind.

8. Bildgebungsvorrichtung nach einem der vorhergehenden Ansprüche, wobei, wenn die Anzahl von auszuführenden Punktionen der Prozedurdaten (162) höher ist als 2, der Selektor (40) die Diffusionsparameter (424) des Teilsatzes von Diffusionsparametern (42) so wählt, dass sie paarweise den gleichen Abstand haben.

9. Bildgebungsvorrichtung nach einem der Ansprüche 1 bis 7, wobei, wenn die Anzahl von auszuführenden Punktionen (N) der Prozedurdaten (162) höher ist als 2, der Selektor (40) einen Medianwert (M) der Diffusionsparameter des Satzes von Diffusionsparametern (36) bestimmt und die Diffusionsparameter ($Dj$), wobei $j$ eine natürliche Ganzzahl zwischen 1 und der Anzahl von auszuführenden Punktionen ($N$) der Prozedurdaten (162) ist, des Teilsatzes von Diffusionsparametern (42) gemäß der Gleichung bestimmt:

$$Dj = Dini + Delta(j - 1),$$

wobei *Dini* die Gleichung erfüllt:

$$Dini = M - min(|M - Dmin|, |M - Dmax|),$$

und Delta die Gleichung erfüllt:

$$Delta = 2(M - Dini)/(N - 1),$$

wobei *Dmin* gleich dem Wert des vom Selektor (40) bestimmten ersten Diffusionsparameters (420) und *Dmax* gleich dem Wert des vom Selektor (40) bestimmten zweiten Diffusionsparameters (426) des Teilsatzes von Diffusionsparametern (42) ist.

10. Bildgebungsvorrichtung nach einem der vorhergehenden Ansprüche, wobei die Punktionstiefe (520), die Punktionsausrichtung (522), der Punktionseintrittspunkt (524) jedes der von der Führung (50 bestimmten Sätze von Punktionsparametern (52) mit den Biopsiedaten (16) eine Punktionsbahn der Biopsienadel definieren, und wobei der Satz von Voraussetzungen (300) der im Speicher (10) gespeicherten Prozedurdaten (162) eine oder mehrere Voraussetzungen enthält, die aus der Gruppe ausgewählt werden, die enthält:

- die Punktionen einer Biopsieprozedur müssen den gleichen Eintrittspunkt (524) in den Tumor (200) haben,
- die Punktionsbahnen sind in gerader Linie und müssen in einem maximalen Punktionswinkel enthalten sein,
- die Punktionstiefe (520) ist geringer als eine maximale Punktionstiefe,
- der Punktionsbereich (526) ist von den Rändern des Tumors (200) um einen vorbestimmten Abstand entfernt,
- wenn der Tumor (200) einen nekrotischen Bereich (500) enthält, dringt die Punktionsbahn nicht in den nekrotischen Bereich (500) ein,
- wenn der Tumor (200) einen Fettgewebebereich enthält, schneidet keiner der Punktionsbereiche (526) den Fettgewebebereich,
- mindestens einer der Eintrittspunkte (524) der Daten von Sätzen von Punktionsparametern (52) wird vorab festgelegt.

11. Bildgebungsvorrichtung nach einem der vorhergehenden Ansprüche, wobei der Speicher (10) außerdem eingerichtet ist, um Punktionsdaten (58) zu empfangen, die einen Satz von Zelldichten enthalten, wobei jede Zelldichte einem der Punktionsbereiche (526) der Daten von Sätzen von Punktionsparametern (52) zugeordnet ist, wobei jede Zelldichte einen Wert hat, der eine Zelldichte des Punktionsbereichs (526) darstellt,
wobei die Bildgebungsvorrichtung (1) außerdem enthält:

- einen Korrelator (60), der eingerichtet ist, um jedem Zelldichtewert, der einem der Punktionsbereiche (526) zugeordnet ist, den Diffusionsparameterwert der Region (544) zuzuordnen, in der der Punktionsbereich (526) im Wesentlichen enthalten ist, und dies für den Satz von Regionen (54), und um daraus Korrelationsdaten (62) zu gewinnen, die Zelldichtewerte und Diffusionsparameterwerte aus dem Satz von Diffusionsparametern (36) einander zuordnen, und
- einen Konstruktor (70), der eingerichtet ist, um Dichtedaten (72) ausgehend vom Verarbeitungsbild (22) und Korrelationsdaten (62) zu bestimmen, wobei die Dichtedaten (72) ein Dichtebild enthalten, von dem jedes Pixel, das einer Region des Schnittbilds entspricht, einer Zelldichte zugeordnet ist, deren Wert in den Korrelationsdaten (62) dem Diffusionsparameterwert eines Pixels des Verarbeitungsbilds (22) entsprechend der Region des Schnittbilds des Pixels zugeordnet ist.

12. Bildgebungsvorrichtung nach Anspruch 11, wobei der Korrelator (60) eine strikt monotone lineare oder nicht lineare Interpolation auf der Basis von Paaren realisiert, die von einem Diffusionsparameterwert und von einem Zelldichtewert gebildet werden, die einem Punktionsbereich (526) zugeordnet sind, und vom Ergebnis der Interpolation die Korrelationsdaten (62) gewinnt.

13. Bildgebungsvorrichtung nach Anspruch 11 oder 12, wobei die Zelldichte der Punktionsdaten und der Dichtedaten (72) einer Gesamtzelldichte oder einer Dichte von Krebszellen entspricht.

14. Bildgebungsvorrichtung nach einem der Ansprüche 11 bis 13, die außerdem eine Schätzvorrichtung enthält, die eingerichtet ist, eine Tumorlast des Tumors (200) auf der Basis der Dichtedaten (72) zu bestimmen.

**Claims**

1. Imaging device comprising:

- a memory (10), arranged to receive

imaging data (12) comprising at least one matrix image (14) derived from a cross-sectional diffusion-weighted magnetic resonance image of a tumour, each pixel of said matrix image (14) being associated with a diffusion parameter of which the value represents the mobility of water molecules within said tumour,
biopsy data (16) comprising needle data (160) defining the dimensions of a puncture made using a biopsy needle, and
procedural data (162) comprising a number of punctures to be performed in a biopsy procedure greater than or equal to 2 and a set of constraints (300) associated with interventional constraints to be met in order to carry out the number of punctures to be performed,

- an oversampler (20), arranged to oversample the matrix image (14) into a processing image (22) of which the spatial resolution is such that each pixel of the processing image (22) corresponds to a region of the substantially square cross-sectional image of which the side has a length less than a needle diameter dimension of the needle data (160);
- a cutter (30) arranged to partition the processing image (22) from the oversampler (20) into a set of regions (32) wherein each region (34)

comprises pixels of the processing image (22) directly adjoining each other at least in twos,
is associated with a diffusion parameter of which the value is substantially equal to the mean of the values of the diffusion parameters associated with its constituent pixels,

is such that

the variance of the values of the diffusion parameters associated with the pixels of the region is less than a given variance value,
corresponds to a portion of the cross-sectional diffusion-weighted magnetic resonance image of said tumour of which the dimensions are greater than or substantially equal to the dimensions of a puncture made using a biopsy needle of the biopsy data (16),
and wherein the diffusion parameters associated with each region (34) of the set of regions (32) form a set of diffusion parameters (36),

- a selector (40), arranged to

determine within the set of diffusion parameters (36) a subset of diffusion parameters (42) comprising as many diffusion parameters as the number of punctures to be performed of the procedural data (162), the subset of diffusion parameters (42) comprising at least one first diffusion parameter (420) selected from the first decile of the diffusion parameter values of the set of diffusion parameters (36), a second diffusion parameter (426) from the last decile of the diffusion parameter values of the set of diffusion parameters (36), and, when the number of punctures to be performed of the procedural data (162) is greater than 2, diffusion parameters (424) of which the respective diffusion parameter values are between the value of the first diffusion parameter (420) and the value of the second diffusion parameter (426),
return a subset of regions (44) derived from the set of regions (32) and wherein each region (46) is associated with a diffusion parameter substantially equal to one of the parameters of the subset of diffusion parameters (42),

- a guide (50), arranged to determine, from the subset of regions (44), data on sets of puncture parameters (52) of which the number is equal to the number of punctures to be performed of the procedural data (162), each set of puncture parameters comprising a puncture depth (520), a puncture orientation (522) and a puncture entry point (524) and defining with the needle data (160) a puncture zone (526) that is substantially included in one of the regions (544) of the subset of regions (54), such that the sets of puncture parameters (52) together meet the set of constraints (300) of the procedural data (162).

2. Imaging device according to claim 1, wherein the oversampler (20) is arranged to oversample the matrix image (14) into the processing image (22) by carrying out an interpolation.

3. Imaging device according to claim 2, wherein the oversampler (20) is arranged to carry out a bicubic interpolation to oversample the matrix image (14) into the processing image (22).

4. Imaging device according to one of the preceding claims, wherein the cutter (30) is arranged to partition the processing image (22) from the oversampler (20) by a super-pixel method.

5. Imaging device according to one of the preceding claims, wherein the number of punctures to be performed in a biopsy procedure of the procedural data (162) stored in the memory (10) is less than or equal to 4.

6. Imaging device according to one of the preceding claims, wherein the selector (40) selects the first diffusion parameter (420) from the second percentile of the diffusion parameter values of the set of diffusion parameters (36), and the second diffusion parameter (426) from the ninety-eighth percentile of the diffusion parameter values of the set of diffusion parameters (36).

7. Imaging device according to one of the preceding claims, wherein the first diffusion parameter (420) and the second diffusion parameter (426) are each equal to the median value of their respective quantile.

8. Imaging device according to one of the preceding claims, wherein when the number of punctures to be performed of the procedural data (162) is greater than 2, the selector (40) selects the diffusion parameters (424) of the subset of diffusion parameters (42) so that they are equidistant from each other in twos.

9. Imaging device according to one of claims 1 to 7, wherein when the number of punctures ($N$) to be performed of the procedural data (162) is greater than 2, the selector (40) determines a median value ($M$) of the diffusion parameters of the set of diffusion parameters (36), and determines the diffusion parameters ($Dj$), j being a natural integer between 1 and the number of punctures ($N$) to be performed of the procedural data (162), of the subset of diffusion parameters (42) according to the equation:

$$Dj=Dini+Delta(j-1),$$

where $Dini$ verifies the equation:

$$Dini=M-min(|M-Dmin|,|M-Dmax|)$$

and $Delta$ verifies the equation:

$$Delta=2(M-Dini)/(N-1),$$

$Dmin$ being equal to the value of the first diffusion parameter (420) determined by the selector (40) and $Dmax$ being equal to the value of the second diffusion parameter (426) of the subset of diffusion parameters (42) determined by the selector (40).

10. Imaging device according to one of the preceding claims, wherein the puncture depth (520), the puncture orientation (522), the puncture entry point (524) of each of the sets of puncture parameters (52) determined by the guide (50) define with the biopsy data (16) a puncture trajectory of the biopsy needle, and wherein the set of constraints (300) of the procedural data (162) stored in the memory (10) comprises one or more constraints selected from the group comprising:

- the punctures of a biopsy procedure must have the same entry point (524) into the tumour (200),
- the puncture trajectories are in a straight line and must be within a maximum puncture angle,
- the puncture depth (520) is less than a maximum puncture depth,
- the puncture zone (526) is spaced from the edges of the tumour (200) by a predetermined distance,
- if the tumour (200) comprises a necrotic zone (500), the puncture trajectory does not penetrate said necrotic zone (500),
- if the tumour (200) comprises a fatty tissue zone, none of the puncture zones (526) intersects said fatty tissue zone,
- at least one of the entry points (524) of the data on sets of puncture parameters (52) is fixed in advance.

11. Imaging device according to one of the preceding claims, wherein the memory (10) is further arranged to receive puncture data (58) comprising a set of cell densities, each cell density being associated with one of the puncture zones (526) of the data on sets of puncture parameters (52), each cell density having a value representing a cell density of said puncture zone (526),
the imaging device (1) further comprising:

- a correlator (60), arranged to associate with each cell density value associated with one of the puncture zones (526) the diffusion parameter value of the region (544) wherein said puncture zone (526) is substantially included, and this for the set of regions (54), and to derive therefrom correlation data (62) associating cell density values and diffusion parameter values of the set of diffusion parameters (36), and

- a constructor (70), arranged to determine density data (72) from the processing image (22) and correlation data (62), the density data (72) comprising a density image of which each pixel, which corresponds to a region of the cross-sectional image, is associated with a cell density of which the value is associated in the correlation (62) data with the diffusion parameter value of a pixel of the processing image (22) corresponding to said region of the cross-sectional image of said pixel.

12. Imaging device according to claim 11, wherein the correlator (60) carries out a strictly monotonic linear or non-linear interpolation based on pairs formed of a diffusion parameter value and of a cell density value associated with a puncture zone (526) and derives the correlation data (62) from the result of said interpolation.

13. Imaging device according to claim 11 or 12, wherein the cell density of the puncture data and the density data (72) corresponds to a total cell density or a cancer cell density.

14. Imaging device according to one of claims 11 to 13, further comprising an estimator arranged to determine a tumour load of the tumour (200) based on the density data (72).

[Fig. 1]

[Fig. 2]

14

200

[Fig. 3]

304

200

306

310

302

300

312

308

[Fig. 4]

22

[Fig. 5]

[Fig. 6]

[Fig. 7]

[Fig. 8]

[Fig. 9]

[Fig. 10]

[Fig. 11]

[Fig. 12]

[Fig. 13]

[Fig. 14]

[Fig. 15]

[Fig. 16]

608

602   604   606

[Fig. 17]

170

[Fig. 18]

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **MATSUMOTO RYUTARO et al.** Needle Insertion Path planning System for Lower Abdominal Insertion Based on CT Images. *International Conférence on Robotics and Biomimetics,* 2018, 182-185
- **PONDMAN KIRSTEN M et al.** MR-Guided Biopsy of the Prostate: An Overview of Techniques and a Systematic Review. *European Urology,* vol. 54 (3), 517-527
- **KEYS, R.** Cubic convolution interpolation for digital image processing. *IEEE transactions on Signal Processing. In Acoustics, Speech, and Signal Processing,* 1981, vol. 29, 1153
- **DUGAD, R. ; AHUJA, N.** A fast scheme for image size change in the compressed domain. *IEEE Transactions on Circuits and Systems for Video Technology,* 2001, vol. 11 (4), 461-474
- **PARK, H ; PARK, Y ; OH, S. K.** LIM-fold image resizing in block-DCT domain using symmetric convolution. *IEEE Transactions on Image Processing,* 2003, vol. 12 (9), 1016-1034
- **WANG, Z. ; CHEN, J. ; HOI, S. C.** Deep learning for image super-resolution: A survey. *arXiv: 1902.06068,* 2019
- Learning a classification model for segmentation. **REN, X ; MALIK, J.** In Proceedings Ninth IEEE International Conférence on Computer Vision. IEEE, Octobre 2003, 10
- **STUTZ, D ; HERMANS, A ; LEIBE, B.** Superpixels: An évaluation of the state-of-the-art. *Computer Vision and Image Understanding,* 2018, vol. 166, 1-27
- **DUAN, L. ; LAFARGE, F.** Image partitioning into convex polygons. *In Proceedings of the IEEE Conférence on Computer Vision and Pattern Recognition,* 2015, 3119-3127
- Evaluation of super-voxel methods for early video processing.". **XU, C ; CORSO, J. J.** In 2012 IEEE conférence on computer vision and pattern récognition. IEEE, Juin 2012, 1202-1209
- **YIN, Y. ; SEDLACZEK, O ; MÜLLER, B. ; WARTH, A. ; GONZÂLEZ-VALLINAS, M ; LAHRMANN, B ; GRABE, N ; KAUCZOR, HU. ; BREUHAHN, K ; VIGNON-CLEMENTEL, IE.** Tumor cell load and heterogeneity estimation from diffusion-weighted MRI calibrated with histological data: an example from lung cancer. *IEEE transactions on medical imaging,* 2018, vol. 37 (1), 35-46